(19) Europäisches Patentamt
European Patent Office
Office européen des brevets

(11) **EP 3 570 756 B1**

(12) **EUROPEAN PATENT SPECIFICATION**

(45) Date of publication and mention
of the grant of the patent:
**17.03.2021 Bulletin 2021/11**

(51) Int Cl.:
***A61B 8/08*** *(2006.01)*   ***A61B 8/00*** *(2006.01)*
***A61B 5/06*** *(2006.01)*

(21) Application number: **18706641.0**

(86) International application number:
**PCT/EP2018/050800**

(22) Date of filing: **15.01.2018**

(87) International publication number:
**WO 2018/134138 (26.07.2018 Gazette 2018/30)**

(54) **SYSTEM FOR IMAGING AND TRACKING INTERVENTIONAL DEVICES**

SYSTEM ZUR BILDGEBUNG UND VERFOLGUNG VON INTERVENTIONELLEN VORRICHTUNGEN

SYSTÈME D'IMAGERIE ET DE SUIVI DE DISPOSITIFS D'INTERVENTION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.01.2017 US 201762448107 P**

(43) Date of publication of application:
**27.11.2019 Bulletin 2019/48**

(73) Proprietor: **Koninklijke Philips N.V.**
**5656 AG Eindhoven (NL)**

(72) Inventors:
• **VAIDYA, Kunal**
**5656 AE Eindhoven (NL)**
• **BHARAT, Shyam**
**5656 AE Eindhoven (NL)**
• **NGUYEN, Man**
**5656 AE Eindhoven (NL)**

(74) Representative: **Philips Intellectual Property &
Standards**
**High Tech Campus 5**
**5656 AE Eindhoven (NL)**

(56) References cited:
**WO-A1-2016/178579   US-A1- 2007 276 243
US-A1- 2013 296 691   US-A1- 2016 324 501**

## Description

Technical Field

The present invention generally relates to ultrasound-guided tracking of interventional devices.

Background

Interventional procedures, which involve inserting catheters, needles and other devices through small incisions in a patient's skin to treat internal conditions, provide a minimally invasive treatment alternative to the traditional open surgical approach. Often the success of the interventional procedure is dependent on image guidance of the interventional devices during the procedure. Image guidance is needed to, for example, locate a treatment site within a patient, direct and track one or more interventional devices to the treatment site, perform procedure at the treatment site, and assess efficacy of treatment.

Fluoroscopy and computed tomography are external imaging modalities that rely on x-ray radiation and injection of contrast dyes for visualization of the tool and internal treatment site for the interventional procedure. While computed tomography provides high resolution and volumetric coverage, it is expensive and its image acquisition can be time consuming. Fluoroscopy provides cheaper real-time imaging of the treatment site, however, the resulting scans often suffer from low resolution and lack of contrast between imaged structures. A mutual drawback of these imaging modalities is that the patient and medical personnel are exposed to radiation, which risks skin or ocular trauma and possibly cancer.

In light of these deficiencies, ultrasound technology, which relies on sound waves, is more frequently being used as a safe, cheaper, and high resolution alternative for guiding interventional procedures. Despite these ultrasound benefits, however, the heterogeneity of imaged tissue (e.g. different organs, tissue interfaces, bony anatomy, etc.) can result in acoustic artifacts (e.g., reverberations, reflections, speckle, shadowing, etc.) in the generated ultrasound images. While acoustic artifacts are often used by clinicians for diagnostic purposes, they can result in the inability to locate or track an interventional tool during the procedure. This can increase the length of the procedure, result in multiple re-entries or path deviations of the interventional device, or result in a failed procedure, all of which increase the clinician's stress and risk discomfort or harm to the patient.

A document US 2013/296691 A1 discloses an electromagnetic needle tracking system that includes a needle assembly, a calibration system and a computing system. The needle assembly includes a needle stylet and a sensor assembly. The sensor assembly includes a sensor that measures position and angular orientation data when placed within an electromagnetic field. The calibration system measures the sensor's position and angular orientation for a known needle tip position and angular orientation within a calibration fixture and calculates a position offset and an angular orientation offset of the sensor relative to the needle tip position and angular orientation. The computing system computes position and angular orientation data of the needle tip by adding the sensor position offset and angular orientation offset to the measured position and angular orientation data, respectively.

Another document WO 2016/178579 A1 relates to a spinal navigation method. The method comprises the steps of providing a MRI, X-ray or CT based two-dimensional image of the spine of a subject and generating an ultrasound two-dimensional image using an ultrasound imaging device on the spine of said subject. Further, the method comprises the steps of matching the ultrasound two-dimensional image to the MRI, X-ray or CT based two-dimensional image, and relating a pre-specified segment of a spinal profile in the MRI, X-ray or CT based two-dimensional image to a corresponding segment in the ultrasound two- dimensional image.

Summary

The invention is defined by the systems of independent claims 1 and 8. Preferred embodiments are defined by the dependent claims. Any disclosed methods are merely exemplary and do not fall within the scope of the present invention.

The present invention generally improves image guidance during interventional procedures by imaging and tracking an interventional device using two or more imaging elements with different field of views. With the different field of views, imaging data obtained from the two or more imaging elements can be processed and/or combined to, for example, reduce image artifacts, confirm positional data of the interventional tool or other objects within the field of view, generate images with larger, combined field of view.

According to certain aspects, imaging systems of the invention include at least two imaging elements, each configured to emit and receive signals corresponding to different field of views of a region of interest. The system further includes an interventional device that includes an elongate body and a sensor located on the elongate body. The sensor is responsive to at least one signal emitted from at least one of the imaging elements. The system further includes at least one processor in communication with the imaging elements and the sensor of the interventional device. The processor is configured to generate an image from signals received by the imaging elements and identify a position of the interventional device using the sensor signal data received from the sensor, in which the sensor signal data corresponds to a signal emitted from at least one of the imaging elements. In certain embodiments, the system may further include a multiplexer that is configured to compile signals from the imaging elements and deliver the compiled signals to the processor.

In further aspects, an identified position of the interventional device is determined by comparing sensor signal data received by the sensor from multiple imaging elements. For example, the sensor signal data may include first sensor signal data corresponding to a first imaging element and second sensor signal data corresponding to a second sensor element. The first and second sensor signal data can then be compared to identify the position of the interventional device. The sensor signal data from each imaging element may include coordinate information for the interventional device. The coordinate information from the sensor data can be compared for enhanced tracking of the interventional device. In some instances, coordinate information from signal data received from various imaging elements is weighted and compared to identify the position of the interventional device. For example, coordinate information from a first signal data (received from a first imaging element) and coordinate information from a second signal data (received from a second imaging element) can be weighted and compared to identify a true position of the interventional device.

In certain embodiments, positions of the imaging elements may be tracked relevant to each other and/or tracked relative to a reference point. For example, the at least two imaging elements may include a first imaging element and a second imaging element, and a position of the first imaging element may be tracked relative to a position of a second imaging element and/or relative to a reference point. The positions of the at least two imaging elements may be tracked, for example, using electromagnetic tracking, optical tracking, and combinations thereof. The imaging elements may be fixed (e.g. in a fixed position) or movable. A fixed imaging element may be placed in a probe holder, or the fixed imaging element may be held in place by adhesive or a band.

The imaging elements may be incorporated into a probe or a patch. Preferably, the imaging elements are ultrasound imaging elements. For example, the imaging element may include one or more ultrasound transducers. The ultrasound transducers may be incorporated into an array.

Any suitable interventional device can be tracked in accordance with the invention. The interventional device may include, for example, an imaging catheter, an atherectomy catheter, an implant delivery catheter, biopsy needle, therapy needle etc. As discussed, the interventional device includes one or more sensors responsive to signals emitted from the imaging elements. In certain embodiments, the sensor may be located at any position on the interventional device that may be useful for tracking the interventional device during the procedure. For example, a preferred location of the sensor is at or proximate to a distal end of the interventional device or at or proximate to a working element of the interventional device (e.g., co-located with an imaging element or ablative element).

According to further aspects, methods are disclosed for identifying a position of an interventional device involve receiving image signals from at least two imaging elements, wherein the imaging elements are configured to transmit and receive image signals from different field of views of a region of interest. An image may then be generated from the received image signals. The method further includes receiving sensor signal data from a sensor located on an interventional device disposed within at least one imaging element's field of view. The sensor signal data corresponds to a signal received from at least one of the imaging elements. The method further involves identifying a position of the interventional device within the generated image based on the sensor signal data.

According to further aspects, systems of the invention for identifying a position of an interventional device further include a processing unit and storage coupled to the processing unit for storing instructions that when executed by the processing unit cause the processing unit to: receive image signals from at least two imaging elements, the imaging elements are configured to transmit and receive image signals from different field of views of a region of interest; generate an image from the received image signals; receive sensor signal data from a sensor located on an interventional device disposed within at least one imaging element's field of view, the sensor signal data corresponds to a signal received from at least one of the imaging elements; and identify a position of the interventional device within the generated image based on the sensor signal data.

Brief Description of the Drawings

FIG. 1 illustrates two or more imaging elements for imaging and tracking interventional devices according to certain embodiments.
FIG. 2 illustrates use of a probe holder in accordance with certain embodiments.
FIG. 3 is a block diagram of an ultrasound imaging system in accordance with the present disclosure.
FIG. 4 illustrates a mechanical position pointer technique in accordance with certain embodiments.
FIG. 5 illustrates use of electromagnetic field generator in accordance with certain embodiments.
FIG. 6 illustrates tracking of an interventional device in a field of view of an imaging element, in accordance with certain embodiments.

Detailed Description

The present invention relates to systems for monitoring an interventional procedure. Systems of the invention include at least two imaging elements, each configured to emit and receive signals corresponding to different fields of view, and an interventional device that includes at least one sensor being responsive to at least one signal emitted from at least one of the imaging elements. The system further includes a processor configured to receive signals from the imaging elements to generate an image and identify a position of the interventional device in the image using the signal data received from the sensor that correspond to the at least one signal emitted from the at least one imaging element.

Systems of the invention are applicable for imaging and tracking devices in a variety of interventional procedures. Interventional procedures may include any procedure in which a physician inserts a device or tool into a patient's body to, e.g., biopsy, monitor, diagnose or treat. Exemplary interventional procedures may include, but are not limited to: arteriovenous malformations, angioplasty, biliary drainage and stenting, catheter embolization, central venous access, chemoembolization, gastrostomy tube insertion, hemodialysis access maintenance, balloon catheterization, needle biopsy, ablation, grafting, thrombolysis, shunting (e.g. transjugular intrahepatic portosystemic shunt), urinary catheterization, uterine catheterization, filter or stent implantation (e.g. vena cava filter). For example, systems and methods of the invention are well-suited for monitoring treatment of cardiovascular disease. When used during a cardiovascular procedure, at least one transducer probe may be positioned, for example, to provide a parasternal view of the heart and another may be positioned, for example, to provide an apical view of the heart. The different views ensure a wide field of view of any interventional tool being inserted within a patient.

In certain embodiments, the interventional device is configured for entry into one or more body lumens, and are imaged by the imaging elements. Various biological lumens include blood vessels, vasculature of the lymphatic and nervous systems, various structures of the gastrointestinal tract including lumen of the small intestine, large intestine, stomach, esophagus, colon, pancreatic duct, bile duct, hepatic duct, lumen of the reproductive tract including the vas deferens, vagina, uterus and fallopian tubes, structures of the urinary tract including urinary collecting ducts, renal tubules, ureter, and bladder, and structures of the head, neck and pulmonary system including sinuses, parotid, trachea, bronchi, and lungs.

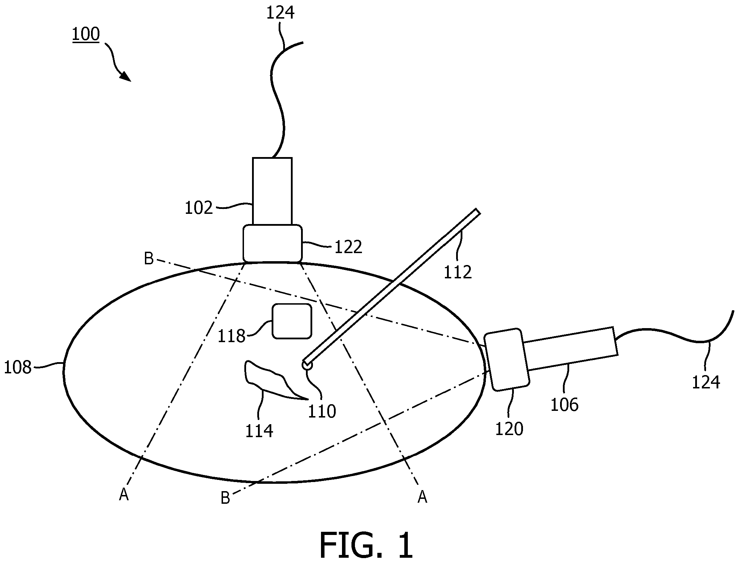

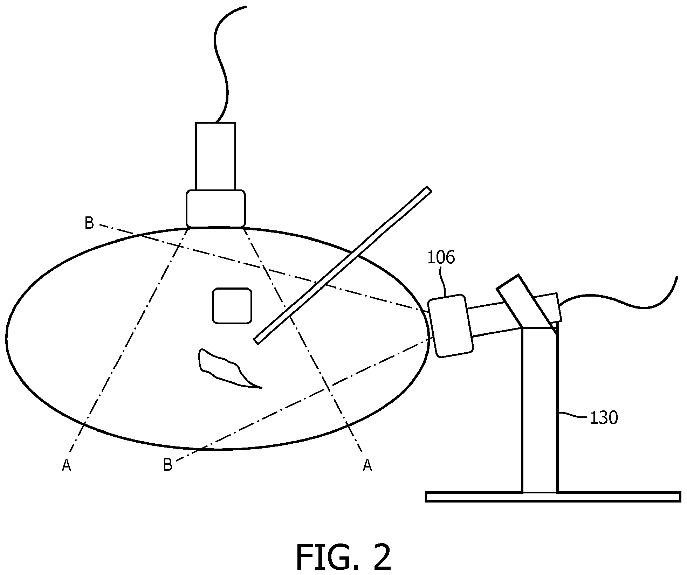

Turning now to the figures, FIG. 1 illustrates an exemplary system 100 of the invention for imaging and tracking an interventional tool 112 as it is directed to a region of interest 114 within a subject or patient 108. While the subject 108 is usually human, it is understood that aspects and embodiments discussed herein are applicable to a wide variety of animals and subjects. The system 100 includes at least two imaging elements 122, 120. In some instances, systems of the invention include 2, 3, 4, 5, or more imaging elements. The imaging elements may be movable, the imaging elements may be fixed or immobile with respect to each other or a reference point, or at least one imaging element may be moveable and at least one imaging element may be fixed. As shown, the imaging elements may be incorporated into imaging probes 102, 106. In certain embodiments, the imaging probes 102, 106 are both hand-held and moveable with respect to each other. In other embodiments, at least one of the probes may be held immobile with respect to the region of interest. FIG. 2 shows imaging probe 106 in a fixed-stand 130. As an alternative to the fixed-stand 130, at least one of the imaging elements may be held against the subject using an adhesive patch or a band. The imaging elements 122, 120 are coupled to wires 124, which connect the imaging elements 122, 120 to one or more processors of an imaging system (described hereinafter). The imaging elements 122, 120 are positioned on a patient 108 in order to image a region of interest 114 and such that the field of view of each imaging element differs from each other. As shown in the figures, the field of view of imaging element 120 is represented by dashed-lines B, and the field of view of imaging element 122 is represented by dashed-lines A. The differing field of views of the imaging elements may overlap as shown, or may be different.

The at least two imaging elements 120, 122 are configured to send imaging signals to and receive imaging signals from the region of interest within their respective field of views or a portion thereof. In certain embodiments, the imaging signals includes acoustic signals. In other embodiments, the imaging signals may be or also include photoacoustic signals. The received imaging signals of the region of interest can be used to generate one or more images. In certain embodiments, the received imaging signals generate a continuous imaging stream of the region of interest in real-time. The imaging elements 120, 122 can also be used to generate separate images of the region of interest 114 within their respective field of views. For example, an image can be generated from the signals received by imaging element 120, and an image can be generated from the signals received by imaging element 122. Additionally, the imaging elements can be used to generate a single large field of view image of the region of interest 114 by co-registering the signals of the imaging elements.

According to certain embodiments, the imaging elements 120, 122 alternate imaging of the region of interest, in which the first imaging element images for a period of time and then the second imaging element images for the period of time. The alternating imaging sequence can occur for a plurality of cycles, and it is understood that length of time each imaging element images may be dependent on the imaging application, determined by the operator, or preprogrammed. In certain embodiments, the alternating imaging of the imaging elements is intermittent, and in other embodiments, the alternating imaging of the imaging elements follows a pattern. The switching rate between imaging elements may be as fast as milli- or microseconds. In certain embodiments, the imaging sequence of the imaging elements may be adjusted based on the presence of artifacts (e.g.

118 of FIG. 1) within the field of view. In addition to the image timing of each imaging element, the direction of the imaging beams of the imaging elements may be controlled, adjusted, or preprogrammed depending on the technical application, positioning of the imaging elements, and/or presence of artifacts (e.g. 118 of FIG. 1). The processing of the signals from the at least two imaging elements 120, 122 is discussed in more detail hereinafter.

According to certain aspects, the imaging elements 120, 122 are ultrasound imaging elements. Imaging element 120 may be the same as or different from imaging element 122. The imaging elements 120, 122 may be incorporated into a probe (as shown), patch, etc. The imaging elements 120, 120 may include one or more ultrasound transducers. The ultrasound transducer may include piezoelectric transducer elements, capacitive micro-machined transducer elements, or any other suitable ultrasound transducer element. In certain embodiments, the imaging element includes a plurality of ultrasound transducers that form an ultrasound array. A variety of transducer arrays may be used for each of the imaging elements, e.g., linear arrays, curved arrays, or phased arrays. Imaging elements may include, for example, a two dimensional array of transducer elements capable of scanning in both elevation and azimuth dimensions for 2D and/or 3D imaging.

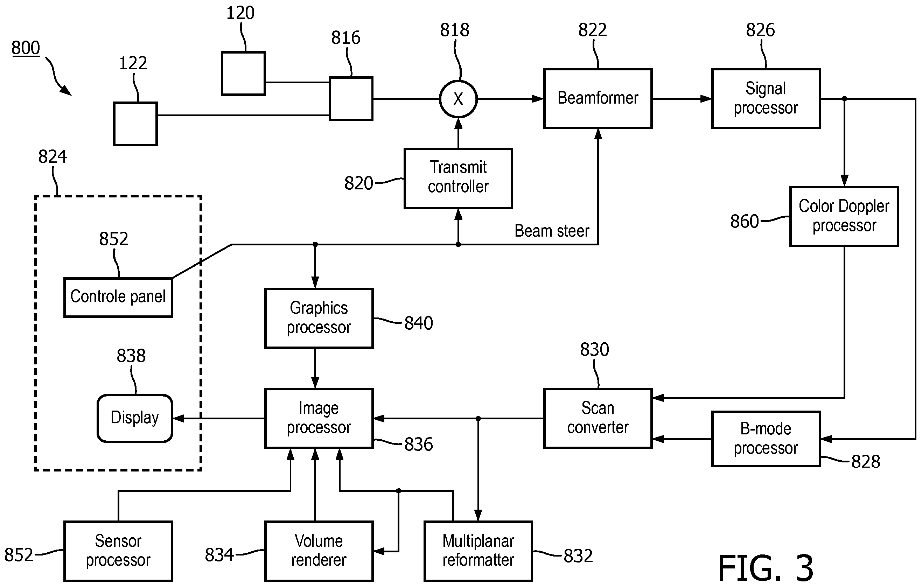

The imaging elements 120, 122 are in communication with an imaging system. The imaging system includes one or more processors that may, e.g., but not limited to, control the imaging elements 120, 122 (e.g. direct imaging), receive imaging data, process imaging data, generate images, provide user interaction, and display the generated images. The processors may include or be the one or more elements described in reference to the ultrasound imaging system of FIG. 3, described below.

FIG. 3 shows an exemplary ultrasound imaging system 800 that may be constructed and used in accordance with the principles of the present disclosure. As described, each imaging element 120, 122 may be configured for transmitting ultrasound waves and receiving echo information. A variety of transducer arrays may be used for each of the imaging elements, e.g., linear arrays, curved arrays, or phased arrays. Imaging elements 120, 122 may include, for example, a two dimensional array of transducer elements capable of scanning in both elevation and azimuth dimensions for 2D and/or 3D imaging. The imaging elements 120, 120 may be coupled to microbeamformers, which may be directly coupled to the transducer or transducer array of the imaging elements (e.g. within the probe or patch) or in an ultrasound system console or base, which may control the transmission and reception of signals of one or more transducers. The imaging elements 120, 122 may be coupled to the ultrasound system base via a multiplexer 816 which may be coupled (via a wired or wireless connection) to a transmit/receive (T/R) switch 818 in the base. The multiplexer may selectively couple one or more of the imaging elements 120, 122 to the base (e.g., to the beamformer 822). The T/R switch 818 may be configured to switch between transmission and reception, e.g., to protect the main beamformer 822 from high energy transmit signals. In some embodiments, the functionality of the T/R switch 818 and other elements in the system may be incorporated in the multiplexer 816. The ultrasound system base typically includes software and hardware components including circuitry for signal processing and image data generation as well as executable instructions for providing a user interface.

The transmission of ultrasonic pulses from transducers of the imaging elements 120, 122 may be directed by the transmit controller 820 coupled to the T/R switch 818 and the beamformer 822, which may receive input from the user's operation of a user interface 824. The user interface 824 may include one or more input devices such as a control panel 852, which may include one or more mechanical controls (e.g., buttons, encoders, etc.), touch sensitive controls (e.g., a trackpad, a touchscreen, or the like), and other known input devices. Another function which may be controlled by the transmit controller 820 is the direction in which beams are steered. Beams may be steered straight ahead from (orthogonal to) the transmission side of the array 812, or at different angles for a wider field of view. The beamformer 822 may combine partially beamformed signals from groups of transducer elements of the individual patches into a fully beamformed signal. The beamformed signals may be coupled to a signal processor 826.

The signal processor 826 can process the received echo signals in various ways, such as bandpass filtering, decimation, I and Q component separation, and harmonic signal separation. The signal processor 826 may also perform additional signal enhancement such as speckle reduction, signal compounding, and noise elimination. The processed signals may be coupled to a B-mode processor 828 for producing B-mode image data. The B-mode processor can employ amplitude detection for the imaging of structures in the body. The signals produced by the B-mode processor 828 may be coupled to a scan converter 830 and a multiplanar reformatter 832. The scan converter 830 is configured to arrange the echo signals in the spatial relationship from which they were received in a desired image format. For instance, the scan converter 830 may arrange the echo signal into a two dimensional (2D) sector-shaped format, or a pyramidal or otherwise shaped three dimensional (3D) format. The multiplanar reformatter 832 can convert echoes which are received from points in a common plane in a volumetric region of the body into an ultrasonic image (e.g., a B-mode image) of that plane, for example as described in U.S. Pat. No. 6,443,896 (Detmer). A volume renderer 834 may generate an image of the 3D dataset as viewed from a given reference point, e.g., as described in U.S. Pat. No. 6,530,885 (Entrekin et al.).

Additionally, the signals from the signal processor 826 may be coupled to a Doppler processor 860, which may be configured to estimate the Doppler shift and generate Doppler image data. The Doppler image data may include color data which is then overlaid with B-mode (or grayscale) image data for display. For example, the Doppler processor may include a Doppler estimator such as an auto-correlator, in which velocity (Doppler frequency) estimation is based on the argument of the lag-one autocorrelation function and Doppler power estimation is based on the magnitude of the lag-zero autocorrelation function. Motion can also be estimated by known phase-domain (for example, parametric frequency estimators such as MUSIC, ESPRIT, etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators. In some examples, the velocity and power estimates may undergo threshold detection to reduce noise, as well as segmentation and post-processing such as filling and smoothing. The velocity and power estimates may then be mapped to a desired range of display colors in accordance with a color map. The color data, also referred to as Doppler image data, is then coupled the scan converter 830 where the Doppler image data is converted to the desired image format and overlaid on the B mode image of the tissue structure containing the blood flow to form a color Doppler image. In certain embodiments, the signal processor 860 processes the signal data from the imaging elements 120, 122 for image registration purposes.

Output (e.g., images) from the scan converter 830, the multiplanar reformatter 832, and/or the volume renderer 834 may be coupled to an image processor 836 for further enhancement, buffering and temporary storage before being displayed on an image display 838. In certain embodiments, the image processor 836 is configured to register images from the processed signal data. A graphics processor 840 may generate graphic overlays for display with the images. These graphic overlays can contain, e.g., standard identifying information such as patient name, date and time of the image, imaging parameters, and the like. For these purposes the graphics processor may be configured to receive input from the user interface 824, such as a typed patient name or other annotations. In some embodiments, one or more functions of at least one of the graphics processor, image processor, volume renderer, and multiplanar reformatter may be combined into an integrated image processing circuitry (the operations of which may be divided among multiple processor operating in parallel) rather than the specific functions described with reference to each of these components being performed by a discrete processing unit. Furthermore, while processing of the echo signals, e.g., for purposes of generating B-mode images or Doppler images are discussed with reference to a B-mode processor and a Doppler processor, it will be understood that the functions of these processors may be integrated into a single processor.

The system 800 may also include or be in communication with a sensor processor 852.

The sensor processor 852 may be included as part of the system 800 or be included in a separate system specific to the interventional device and in communication with system 800. The sensor processor 852 receives sensor signals from the interventional device being tracked by the system 800. The sensor signals are then communicated to the image processor 836, where a position of the interventional device is determined within generated images. The tracking of the interventional device using sensor data is described in more detail hereinafter.

The imaging elements 120, 122 may be used to image a region of interest 114 and guide an interventional tool 112 to the region of interest 114. The region of interest 114 may be a region within the patient that requires monitoring and/or an interventional procedure. For example, the region of interest may be a location within a blood vessel that requires intraluminal imaging, ablative therapy, stent placement, etc. In another example, the region of interest may be tissue that requires biopsy. In another example, the region of interest may be fetus within the maternal womb, or a specific feature on the fetus.

The imaging data from the imaging elements may be co-registered using any known image registration techniques to create a combined image with a large field of view. In certain aspects, the images from the imaging elements 120, 122 are registered using image fusion. In other embodiments, systems of the invention are able to register the imaging signals from imaging elements 120, 122 to create the large field of view image using the known orientation of imaging elements 120, 122 as guidance. In certain embodiments, the known orientation includes knowledge of the orientation at least one imaging element 120 with respect to the orientation of another imaging element 122 and/or reference point. The reference point may be, for example, an immobile object within the field of view of the imaging elements. The reference point may also be, for example, an exterior known location in the tracking coordinate system in which the imaging elements are tracked. The known orientations of the imaging elements reduces processing required to co-register received signals from the imaging elements. A particular benefit of tracking the orientations of the imaging elements 120, 122 is that systems of the invention can dynamically register the images in real-time while at least one imaging element 120 is moving with respect to the other imaging element 122. The images acquired from the two or more imaging elements may be reconstructed independently and registered to provide visualization of the positional sensor 110 on the interventional tool 112.

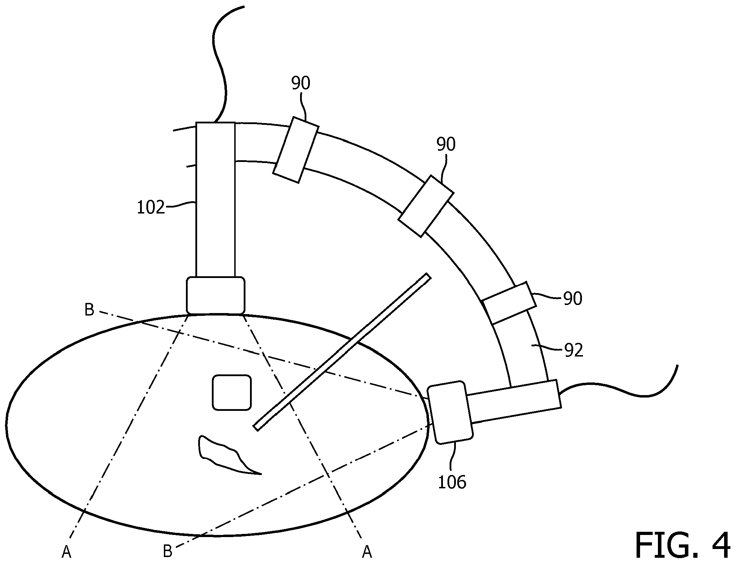

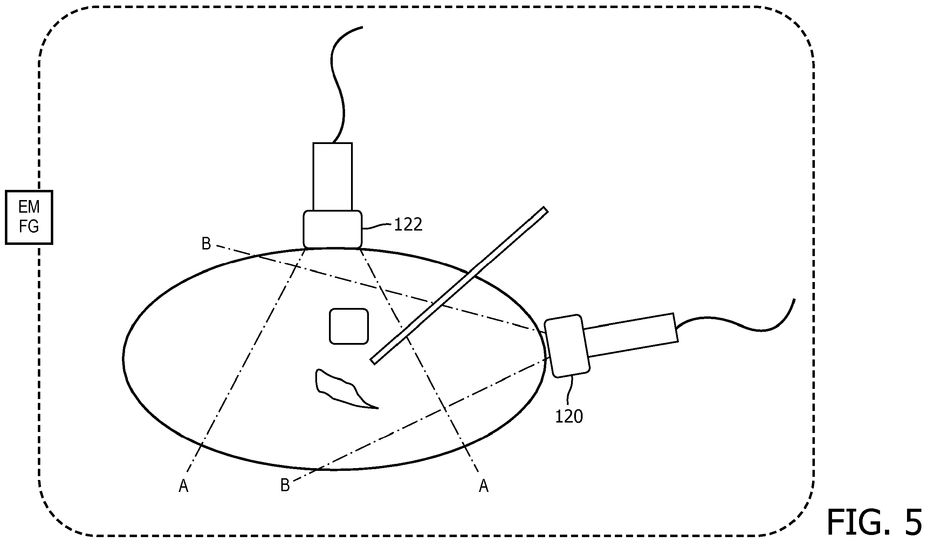

Any suitable technique for determining and monitoring the orientation of the imaging elements 122, 120 and/or reference point may be used. In certain embodiments, the orientation is determined and monitored using electromagnetic tracking. Electromagnetic tracking systems generally include a field generator, which uses coils to generate a magnetic field and establish a coordinate space. The imaging elements, probe and reference point within the magnetic field may include one or more sensors. The sensors may include coils that react to the magnetic field. The reaction of the sensors may be recorded, and the resulting data can provide the orientation and positioning of the sensors, thereby providing the position and orientation of the imaging elements, probes, and/or reference point. As shown in FIG. 5, the imaging elements 122, 120, probes, and/or reference are within an electromagnetic field generated by one or more electromagnetic field generator (EM FG). In other embodiments, the orientation is determined and monitored using optical tracking, in which the imaging elements, probes and/or reference point include one or more optical markers that are detectable by an optical camera. The optical markers may be passive (e.g. reflective) or active (e.g. emitting light). The optical camera scans the imaging elements, probes, and/or reference point with the optical markers, and the resulting imaging are processed to identify and calculate the marker position and orientation, which can be determinative of the position and orientation of the imaging elements, probes, and/or reference point. In other embodiments, mechanical positon pointers are used to determine and monitor the orientation of the imaging elements, probes, and/or reference point. Mechanical position pointer techniques include mechanical digitizers that include a mechanical arm with encoded joints, which can be tracked with respect to a known starting point. FIG. 4 illustrates a mechanical position pointer technique, showing an arm 92 with joints 90 coupled to imaging probes 102, 106. MicroScribe™ is a known mechanical position pointer technology that can be leveraged in aspects of the invention.

Tracking the position and the orientation of the imaging elements can be used to determine the x, y, and/or z coordinates of generated images. The coordinates can then be used to register the images. The registered images can be shown, for example, on a monitor coupled to the imaging system.

According to certain aspects, the two or more imaging elements 120, 122 are configured to image, track, and guide one or more interventional tools 112 to the region of interest 114, as shown in FIG. 1. Typical interventional tools include, for example, guidewires, guide catheters or sheaths, delivery catheters, ablation catheters, imaging catheters, catheter sheaths, needles, and implantable devices (sensors, stents, filters, etc.). The interventional tool 112 includes one or more positional sensors 110. The positional sensor 110 is coupled to one or more wires (not shown) that extend the length of the interventional tool 112 and connect to a processor of the imaging system. The positional sensor 110 may be directly connected to the same processor of the imaging system as the imaging elements 120, 122, or a different processor that is in communication with the processor connected to the imaging elements 120, 122. The positional sensor 110 is configured to receive or transmit signals that are determinative of the position of the positional sensor 110 and thus the interventional tool 112.

In certain embodiments, the sensor may be located at any position on the interventional device that may be useful for tracking the interventional device during the procedure. For example, a preferred location of the sensor is at or proximate to a distal end of the interventional device or at or proximate to a working element of the interventional device (e.g co-located with an imaging element or ablative element).

According to certain aspects, the positional sensor 110 of the interventional tool 112 is configured to receive signals transmitted from the imaging elements 120, 122. For example, the imaging elements 120, 122 transmit imaging signals as described above, and the positional sensor 110 passively listens to the signals transmitted from the imaging elements 120, 122. The received signals from the imaging elements 122, 120 by the positional sensor 110 can be used to determine the position of the positional sensor 110, and thus the position of the interventional tool 112 within the generated image. In certain embodiments, the positional sensor 110 is configured to perform one or more other functions in addition to passively receiving signals of the imaging elements 120, 122 and transmitting the received signal data to its connected processor. The one or more other functions may include, for example, pressure sensing, flow sensing and/or imaging.

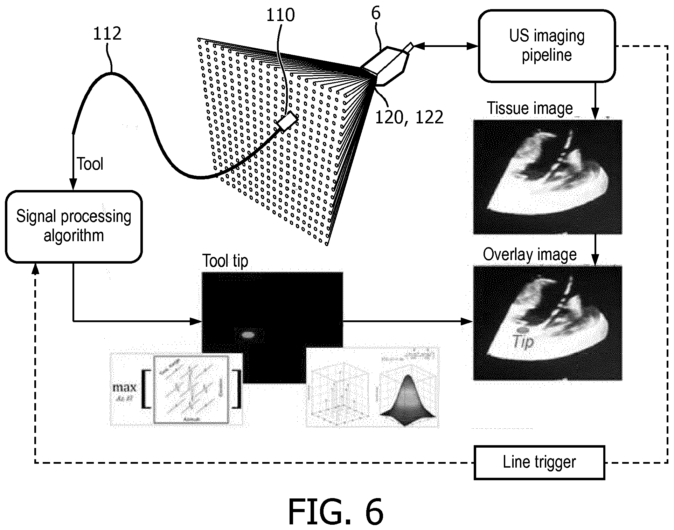

FIG. 6 illustrates a method for tracking of the positional sensor 110 of an interventional tool 112 that is within the field of view of an imaging element 122 of probe 106. While shown with respect to one imaging element, it is understood that the same technology can be applied during imaging with any one of the imaging elements120, 122. As shown, the imaging element 122 is configured to generate 3D images, however it is understood that the imaging element may be configured to generate 1D or 2D images. The positional sensor 110 is configured to receive signals from imaging element 122. The positional sensor 110 and the imaging element 122 are connected to and in communication with an imaging system (e.g. the same processing system or separate processing systems in communication with each other). In order to determine the position of the positional sensor 110 (and thus the interventional tool 112), the imaging element 122 sends and receives signals to generate an image of a region of interest. When the signals are transmitted for imaging, a trigger is sent to the imaging system and/or the positional sensor 110 that indicates when the signal was sent (e.g. starts the clock for a particular signal at zero). The transmitted signal is then received by the positional sensor 110, and time delay between when the signal was transmitted and when the signal was received (e.g. the time of flight of the signal) is used to determine the position of the positional sensor 110 within the imaging beam. That is, the time from beam emission to reception by the positional sensor indicates the depth of the positional sensor 110 within the imaging beam. This can be repeated for a plurality of imaging signals for real-time tracking of the positional sensor and thus the interventional tool in images. The plurality of imaging signals may be sent from a plurality of apertures on the imaging element (e.g. a plurality of transducers on a matrix transducer array) or a single aperture from the imaging element. The position of the imaging beam that yields the highest amplitude sensed at the positional sensor's location corresponds to the lateral (or angular, depending on the imaging geometry) location of the positional sensor. This process is described in more detail in U.S. Patent 9282946.

In addition to technique described above and shown in FIG. 5, it is also contemplated that the location of the positional sensor is tracked based on signals emitted from the positional sensor 110 and received by the two or more imaging elements 120, 122. For example, the positional sensor may send a signal that is received by an imaging element 120, 122, and the time delay between the signal's transmission and receipt is indicative of the positional depth of the positional sensor within the field of view of the imaging elements 120, 122. This process is described in more detail in U.S. Patent Nos. 7,270,684 and 9282946.

Other techniques for determining the position a positional sensor within an image are described in more detail in U.S. Patent Nos. 7,270,684, 7,529,393, and 7796789.

The above-described techniques for identifying a positional sensor 110 within a field of view of an imaging element may be performed by one or more of the imaging elements 120, 122. Ideally, the techniques are continuously repeated by one or more imaging elements 120, 122 to provide real-time tracking of the positional sensor 110 within the registered images generated by the imaging elements 120, 122. With the location of the positional sensor 110 determined by at least one of the imaging elements 120, 122, its location can be registered to the fused image generated from the imaging elements 120, 120. For example, the location of the positional sensor 110 can be overlaid in the registered image from the imaging elements for enhanced visualization of the interventional tool 112. A graphical element is used to show the positional sensor 110 in the resulting image on the monitor.

In preferred embodiments, the location of the positional sensor 110 within a certain time frame is identified and confirmed by at least two of the imaging elements 122, 120. That is, the coordinate location of the positional sensor 110 as determined by imaging elements 122, 120 should be the same or substantially in the registered image, and the complementary tracking can be used to ensure accuracy of the tracking. In some instances, the determined location of positional sensor 110 may differ slightly with respect to the different imaging elements 122, 120. In such instances, the results may be averaged if the results are spatially within a pre-defined threshold. The averaged position of the positional 110 sensor may then be shown in a generated image from the combined image data of the imaging elements 120, 122.

According to further aspects, a tracking history of the location of the positional sensor 110 can be maintained from one or more of the imaging elements 120, 122. In one embodiment, the tracking history of the positional sensor 110 is used to determine whether there is any conflict between the positional determinations reported by the signals sent from the two or more imaging elements. If there is any conflict, the history can be used to resolve the conflict. For example, data from the imaging elements 120, 122 and positional sensor may indicate two different coordinates A, B of the positional sensor within a registered image. To resolve this conflict, systems of the invention may review a temporal tracking history of the positional sensor 110 may show a defined path of the positional sensor 110 in the co-registered images. The coordinate that most closely follows the defined path may be accepted as accurate, and the other coordinate may be disregarded as an outlier. In certain embodiments, a predefined threshold is used to determine whether an identified coordinate of the positional sensor is accurate or an outlier (e.g. within 5% of defined path of the positional sensor).

Another embodiment of the invention uses a weighted system to resolve contradicting locations of the positional sensor 110 based on signals received from different imaging elements 120, 122. For example, in the case that the tracking results provide contradicting locations, weighting can be assigned to each imaging element 120, 122 to calculate a final or corrected position of the positional sensor 110. Factors contributing to the weighting include the presence of occlusion, reverberation, aberration or the subjective opinion of the sonographer and/or surgeon, who may have an estimate of his/her own. The final tracked location (X,Y,Z) can be calculated as

$$X = w1 * x1 + w2 * x2 + \ldots$$

$$Y = w1 * y1 + w2 * y2 + \ldots$$

$$Z = w1 * z1 + w2 * z2 + \ldots$$

where (x1,y1,z1) and (x2,y2,z2) are the locations of the sensor tracked by imaging elements 120, 122 respectively. W1, w2, .... are the weights with a sum of 1. For example, if the sensor is in the shadowing region of 1 probe due to the occlusion, a weight of 0 can be assigned to that probe and the final tracked location can be determined by other probe(s).

It is understood that the positional sensor may not be within the field of view of at least one of the imaging elements 120, 122. While this scenario does not provide for confirmatory locales of the positional sensor, the surgeon or sonographer will still benefit from the large field of view generated from the registered image from the at least two imaging elements.

By using at least two imaging elements 120, 122, with different field of views A, B, systems of the invention are able to accurately image, track and guide at least one interventional device via its positional sensor 110 despite the presence of artifacts 118 within the patient that can obstruct the image of either imaging elements 120, 122. For example and as shown in FIG. 1, the region of interest 114 is in close proximity to an artifact 118 that at least partially obstructs imaging of the path of the interventional device 112 as well as at least partially obstructs the region of interest 114. Using both imaging elements 120, 122, the interventional tool 112 can be tracked without obstruction because imaging element 120 is able to image the intervention tool at locales where the region of interest 114 is obstructed by artifact 118. Additionally, image quality and tracking can be enhanced if at least one of the imaging elements is closer to the region of interest and/or interventional tool 112. For example, image quality may be dependent on depth, and at least one of the probes may be closer to the region of interest and/or interventional tool 112. As shown in FIG. 1, imaging element 122 is closer to the region of interest 114, and thus able to provide a clearer view of the region of interest 114 than imaging element 120 at the locales where the region of interest is not obstructed by artifact 118.

Systems and methods of the invention may be implemented using software, hardware, firmware, hardwiring, or combinations of any of these. Features implementing functions can also be physically located at various positions, including being distributed such that portions of functions are implemented at different physical locations (e.g., imaging apparatus in one room and host workstation in another, or in separate buildings, for example, with wireless or wired connections).

As discussed above, systems of the invention may rely on one or more processors to execute the steps of the invention. Processors suitable for the execution of computer program include, by way of example, both general and special purpose microprocessors, and any one or more processor of any kind of digital computer. Generally, a processor will receive instructions and data from a read-only memory or a random access memory or both. The essential elements of computer are a processor for executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. Information carriers suitable for embodying computer program instructions and data include all forms of non-volatile memory, including by way of example semiconductor memory devices, (e.g., EPROM, EEPROM, solid state drive (SSD), and flash memory devices); magnetic disks, (e.g., internal hard disks or removable disks); magneto-optical disks; and optical disks (e.g., CD and DVD disks). The processor and the memory can be supplemented by, or incorporated in, special purpose logic circuitry.

To provide for interaction with a user, the subject matter described herein can be implemented on a computer having an I/O device, e.g., a CRT, LCD, LED, or projection device for displaying information to the user and an input or output device such as a keyboard and a pointing device, (e.g., a mouse or a trackball), by which the user can provide input to the computer. Other kinds of devices can be used to provide for interaction with a user as well. For example, feedback provided to the user can be any form of sensory feedback, (e.g., visual feedback, auditory feedback, or tactile feedback), and input from the user can be received in any form, including acoustic, speech, or tactile input.

The subject matter described herein can be implemented in a computing system that includes a back-end component (e.g., a data server), a middleware component (e.g., an application server), or a front-end component (e.g., a client computer having a graphical user interface or a web browser through which a user can interact with an implementation of the subject matter described herein), or any combination of such back-end, middleware, and front-end components. The components of the system can be interconnected through network by any form or medium of digital data communication, e.g., a communication network. Examples of communication networks include cell network (e.g., 3G or 4G), a local area network (LAN), and a wide area network (WAN), e.g., the Internet.

The subject matter described herein can be implemented as one or more computer program products, such as one or more computer programs tangibly embodied in an information carrier (e.g., in a non-transitory computer-readable medium) for execution by, or to control the operation of, data processing apparatus (e.g., a programmable processor, a computer, or multiple computers). A computer program (also known as a program, software, software application, app, macro, or code) can be written in any form of programming language, including compiled or interpreted languages (e.g., C, C++, Perl), and it can be deployed in any form, including as a stand-alone program or as a module, component, subroutine, or other unit suitable for use in a computing environment. Systems and methods of the invention can include instructions written in any suitable programming language known in the art, including, without limitation, C, C++, Perl, Java, ActiveX, HTML5, Visual Basic, or JavaScript.

A computer program does not necessarily correspond to a file. A program can be stored in a portion of file that holds other programs or data, in a single file dedicated to the program in question, or in multiple coordinated files (e.g., files that store one or more modules, subprograms, or portions of code). A computer program can be deployed to be executed on one computer or on multiple computers at one site or distributed across multiple sites and interconnected by a communication network.

A file can be a digital file, for example, stored on a hard drive, SSD, CD, or other tangible, non-transitory medium. A file can be sent from one device to another over network (e.g., as packets being sent from a server to a client, for example, through a Network Interface Card, modem, wireless card, or similar).

Writing a file according to the invention involves transforming a tangible, non-transitory computer-readable medium, for example, by adding, removing, or rearranging particles (e.g., with a net charge or dipole moment into patterns of magnetization by read/write heads), the patterns then representing new collocations of information about objective physical phenomena desired by, and useful to, the user. In some embodiments, writing involves a physical transformation of material in tangible, non-transitory computer readable media (e.g., with certain optical properties so that optical read/write devices can then read the new and useful collocation of information, e.g., burning a CD-ROM). In some embodiments, writing a file includes transforming a physical flash memory apparatus such as NAND flash memory device and storing information by transforming physical elements in an array of memory cells made from floating-gate transistors. Methods of writing a file are well-known in the art and, for example, can be invoked manually or automatically by a program or by a save command from software or a write command from a programming language.

Suitable computing devices typically include mass memory, at least one graphical user interface, at least one display device, and typically include communication between devices. The mass memory illustrates a type of computer-readable media, namely computer storage media. Computer storage media may include volatile, nonvolatile, removable, and non-removable media implemented in any method or technology for storage of information, such as computer readable instructions, data structures, program modules, or other data. Examples of computer storage media include RAM, ROM, EEPROM, flash memory, or other memory technology, CD-ROM, digital versatile disks (DVD) or other optical storage, magnetic cassettes, magnetic tape, magnetic disk storage or other magnetic storage devices, Radiofrequency Identification tags or chips, or any other medium which can be used to store the desired information and which can be accessed by a computing device.

**Claims**

1. An imaging system (100, 800), the system comprising
   at least two imaging elements (120, 122), each configured to emit and receive image signals corresponding to different fields of views (A, B);
   an interventional device (112) comprising an elongate body and a sensor (110) located on the elongate body, the sensor (110) being responsive to an image signal emitted from at least one of the imaging elements (120, 122); and
   a processor in communication with the imaging elements (120, 122) and the interventional device (112), the processor configured to generate an image from image signals and identify a position of the interventional device (112) in the image using sensor signal data received from the sensor (110), the sensor signal data corresponding to the image signal emitted from the at least one imaging element (120, 122).

2. The system of claim 1, wherein the at least two imaging elements (120, 122) comprise a first imaging element and a second imaging element and a position of the first imaging element is tracked relative to a position of the second imaging element.

3. The system of claim 2, wherein the first imaging element is further tracked relative to a reference point.

4. The system of claim 1, wherein positions of the at least two imaging elements (120, 122) are tracked using a technique selected from the group consisting of electromagnetic tracking, optical tracking, and combinations thereof.

5. The system of claim 1, wherein the sensor signal data includes first sensor signal data corresponding to a first imaging element and second sensor signal data corresponding to a second sensor element, and the first and second sensor signal data are compared to identify the position of the interventional device (112).

6. The system of claim 5, wherein coordinate information from the first and second signal data is weighted and compared to identify the position of the interventional device (112).

7. The system of claim 1, wherein at least one of the imaging elements comprises an ultrasound transducer.

8. A system for identifying a position of an interventional device (112), the system comprising
   a processing unit; and
   storage coupled to said processing unit for storing instructions that when executed by the processing unit cause the processing unit to:

receive image signals from at least two imaging elements (120, 122), wherein each imaging element is configured to transmit and receive image signals that correspond to different fields of view (A, B) of a region of interest; generate an image from the image signals; receive sensor signal data from a sensor (110), the sensor (110) being responsive to an image signal emitted from at least one of the imaging elements (120, 122) and the sensor (110) being located on an interventional device that is within a field of view (A, B) of at least one imaging element, the sensor signal data corresponding to a signal emitted from the at least one imaging element; and identify a position of the interventional device (112) within the image based on the sensor signal data.

9. The system of claim 8, wherein the received signals from the imaging elements (120, 122) are co-registered to generate the image.

10. The system of claim 8, wherein the imaging elements comprise a first imaging element and a second imaging element, and a position of the first imaging element is tracked relative to a position of the second imaging element.

11. The system of claim 10, wherein the first imaging element is further tracked relative to a reference point.

12. The system of claim 9, wherein positions of the at least two transducer arrays are tracked using electromagnetic tracking or optical tracking.

13. The system of claim 8, wherein the sensor signal data includes first sensor signal data corresponding to a first imaging element and second sensor signal data corresponding to a second sensor element, and the first and second sensor signal data are compared to identify the position of the interventional device.

14. The system of claim 8, wherein coordinate information from the first and second signal data is weighted and compared to identify the position of the interventional device.

15. The system of claim 8, wherein at least one of the imaging elements (120, 122) comprises an ultrasound transducer.

**Patentansprüche**

1. Bildgebungssystem (100, 800), wobei das System umfasst

mindestens zwei Abbildungselemente (120, 122), die jeweils so konfiguriert sind, dass sie Bildsignale senden und empfangen, die unterschiedlichen Sichtfeldern (A, B) entsprechen; eine interventionelle Vorrichtung (112), umfassend einen länglichen Körper und einen Sensor (110), der an dem länglichen Körper angeordnet ist, wobei der Sensor (110) auf ein Bildsignal anspricht, das von mindestens einem der Abbildungselemente (120, 122) emittiert wird; und

einen Prozessor in Kommunikation mit den Abbildungselementen (120, 122) und der interventionellen Vorrichtung (112), wobei der Prozessor konfiguriert ist, um ein Bild aus Bildsignalen zu erzeugen und eine Position der interventionellen Vorrichtung (112) in dem Bild unter Verwendung der empfangenen Sensorsignaldaten zu identifizieren, die von dem Sensor (110) empfangen werden, wobei die Sensorsignaldaten dem von dem mindestens einen Abbildungselement (120, 122) emittierten Bildsignal entsprechen.

2. System nach Anspruch 1, wobei die mindestens zwei Abbildungselemente (120, 122) ein erstes Abbildungselement und ein zweites Abbildungselement umfassen, und eine Position des ersten Abbildungselements relativ zu einer Position des zweiten Abbildungselements verfolgt wird.

3. System nach Anspruch 2, wobei das erste Abbildungselement weiter relativ zu einem Referenzpunkt verfolgt wird.

4. System nach Anspruch 1, wobei die Positionen der mindestens zwei Abbildungselemente (120, 122) unter Verwendung einer Technik verfolgt werden, die aus der Gruppe ausgewählt ist, die aus elektromagnetischer Verfolgung, optischer Verfolgung und Kombinationen davon besteht.

5. System nach Anspruch 1, wobei die Sensorsignaldaten erste Sensorsignaldaten umfassen, die einem ersten Abbildungselement entsprechen, und zweite Sensorsignaldaten, die einem zweiten Sensorelement entsprechen, und die ersten und zweiten Sensorsignaldaten verglichen werden, um die Position der interventionellen Vorrichtung (112) zu identifizieren.

6. System nach Anspruch 5, wobei Koordinateninformationen aus den ersten und zweiten Signaldaten gewichtet und verglichen werden, um die Position der interventionellen Vorrichtung (112) zu identifizieren.

**7.** System nach Anspruch 1, wobei mindestens eines der Abbildungselemente einen Ultraschallwandler umfasst.

**8.** System zum Identifizieren einer Position einer interventionellen Vorrichtung (112), wobei das System umfasst
eine Verarbeitungseinheit; und
Speicher, der mit der Verarbeitungseinheit gekoppelt ist, um Anweisungen zu speichern, die bei Ausführung durch die Verarbeitungseinheit bewirken, dass die Verarbeitungseinheit:

Empfangen von Bildsignalen von mindestens zwei Abbildungselementen (120, 122), wobei jedes Abbildungselement konfiguriert ist, um Bildsignale zu senden und zu empfangen, die unterschiedlichen Sichtfeldern (A, B) eines interessierenden Bereichs entsprechen;
Erzeugen eines Bildes aus den Bildsignalen;
Empfangen von Sensorsignaldaten von einem Sensor (110), wobei der Sensor (110) auf ein Bildsignal anspricht, das von mindestens einem der Abbildungselemente (120, 122) emittiert wird, und der Sensor (110) sich auf einer interventionellen Vorrichtung befindet, die innerhalb eines Sichtfeldes (A, B) von mindestens einem Abbildungselement ist, entsprechen die Sensorsignaldaten einem von mindestens einem Abbildungselement emittierten Signal; und
Identifizieren einer Position der interventionellen Vorrichtung (112) innerhalb des Bildes basierend auf den Sensorsignaldaten.

**9.** System nach Anspruch 8, wobei die empfangenen Signale von den Abbildungselementen (120, 122) gemeinsam registriert werden, um das Bild zu erzeugen.

**10.** System nach Anspruch 8, wobei die Abbildungselemente ein erstes Abbildungselement und ein zweites Abbildungselement umfassen, und eine Position des ersten Abbildungselements relativ zu einer Position des zweiten Abbildungselements verfolgt wird.

**11.** System nach Anspruch 10, wobei das erste Abbildungselement weiter relativ zu einem Referenzpunkt verfolgt wird.

**12.** System nach Anspruch 9, wobei die Positionen der mindestens zwei Wandleranordnungen unter Verwendung von elektromagnetischer Verfolgung oder optischer Verfolgung verfolgt werden.

**13.** System nach Anspruch 8, wobei die Sensorsignaldaten erste Sensorsignaldaten umfassen, die einem ersten Abbildungselement entsprechen, und zweite Sensorsignaldaten, die einem zweiten Sensorelement entsprechen, und die ersten und zweiten Sensorsignaldaten verglichen werden, um die Position der interventionellen Vorrichtung zu identifizieren.

**14.** System nach Anspruch 8, wobei Koordinateninformationen aus den ersten und zweiten Signaldaten gewichtet und verglichen werden, um die Position der interventionellen Vorrichtung zu identifizieren.

**15.** System nach Anspruch 8, wobei mindestens eines der Abbildungselemente (120, 122) einen Ultraschallwandler umfasst.

**Revendications**

**1.** Système d'imagerie (100, 800), le système comprenant
au moins deux éléments d'imagerie (120, 122), chacun configuré pour émettre et recevoir des signaux d'image correspondant à différents champs de vue (A, B) ;
un dispositif d'intervention (112) comprenant un corps allongé et un capteur (110) situé sur le corps allongé, le capteur (110) étant sensible à un signal d'image émis par au moins l'un des éléments d'imagerie (120, 122); et
un processeur en communication avec les éléments d'imagerie (120, 122) et le dispositif d'intervention (112), le processeur étant configuré pour générer une image à partir de signaux d'image et identifier une position du dispositif d'intervention (112) dans l'image en utilisant les données des signaux de capteur reçues du capteur (110), les données des signaux de capteur correspondant au signal d'image émis par l'au moins un élément d'imagerie (120, 122).

**2.** Système selon la revendication 1, dans lequel les au moins deux éléments d'imagerie (120, 122) comprennent un premier élément d'imagerie et un second élément d'imagerie et une position du premier élément d'imagerie est suivie par rapport à une position du second élément d'imagerie.

**3.** Système selon la revendication 2, dans lequel le premier élément d'imagerie est en outre suivi par rapport à un point de référence.

**4.** Système selon la revendication 1, dans lequel les positions des au moins deux éléments d'imagerie (120, 122) sont suivies en utilisant une technique choisie dans le groupe constitué par le suivi électromagnétique, le suivi optique, et leurs combinaisons.

**5.** Système selon la revendication 1, dans lequel les données des signaux de capteur comprennent des premières données de signaux de capteur correspondant à un premier élément d'imagerie et des secondes données de signaux de capteur correspondant à un second élément de capteur, et les première et seconde données des signaux de capteur sont comparées pour identifier la position du dispositif d'intervention (112).

6. Système selon la revendication 5, dans lequel les informations de coordonnées provenant des première et seconde données des signaux sont pondérées et comparées pour identifier la position du dispositif d'intervention (112).

7. Système selon la revendication 1, dans lequel au moins l'un des éléments d'imagerie comprend un transducteur à ultrasons.

8. Système pour identifier une position d'un dispositif d'intervention (112), le système comprenant une unité de traitement; et
une unité de stockage couplée à ladite unité de traitement pour stocker des instructions qui, lorsqu'elles sont exécutées par l'unité de traitement, amènent l'unité de traitement à:

> recevoir des signaux d'image d'au moins deux éléments d'imagerie (120, 122), où chaque élément d'imagerie est configuré pour transmettre et recevoir des signaux d'image qui correspondent à différents champs de vue (A, B) d'une région d'intérêt;
> générer une image à partir des signaux d'image;
> recevoir des données de signaux de capteur d'un capteur (110), le capteur (110) étant sensible à un signal d'image émis par au moins l'un des éléments d'imagerie (120, 122) et le capteur (110) étant situé sur un dispositif d'intervention qui est à l'intérieur d'un champ de vue (A, B) d'au moins un élément d'imagerie, les données des signaux de capteur correspondant à un signal émis par l'au moins un élément d'imagerie; et
> identifier une position du dispositif d'intervention (112) à l'intérieur de l'image sur la base des données des signaux de capteur.

9. Système selon la revendication 8, dans lequel les signaux reçus des éléments d'imagerie (120, 122) sont co-recalés pour générer l'image.

10. Système selon la revendication 8, dans lequel les éléments d'imagerie comprennent un premier élément d'imagerie et un second élément d'imagerie, et une position du premier élément d'imagerie est suivie par rapport à une position du second élément d'imagerie.

11. Système selon la revendication 10, dans lequel le premier élément d'imagerie est en outre suivi par rapport à un point de référence.

12. Système selon la revendication 9, dans lequel les positions des au moins deux réseaux de transducteurs sont suivies en utilisant un suivi électromagnétique ou un suivi optique.

13. Système selon la revendication 8, dans lequel les données des signaux du capteur comprennent des premières données de signaux de capteur correspondant à un premier élément d'imagerie et des secondes données de signaux de capteur correspondant à un second élément de capteur, et les première et seconde données des signaux de capteur sont comparées pour identifier la position du dispositif d'intervention.

14. Système selon la revendication 8, dans lequel les informations de coordonnées provenant des première et seconde données de signaux sont pondérées et comparées pour identifier la position du dispositif d'intervention.

15. Système selon la revendication 8, dans lequel au moins l'un des éléments d'imagerie (120, 122) comprend un transducteur à ultrasons.

FIG. 1

EP 3 570 756 B1

FIG. 2

FIG. 3

FIG. 4

EP 3 570 756 B1

FIG. 5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013296691 A1
- WO 2016178579 A1
- US 6443896 B, Detmer
- US 6530885 B, Entrekin

- US 9282946 B
- US 7270684 B
- US 7529393 B
- US 7796789 B